(19) Europäisches Patentamt
European Patent Office
Office européen des brevets

(11) Veröffentlichungsnummer: **0 176 928**
**A2**

(12) # EUROPÄISCHE PATENTANMELDUNG

(21) Anmeldenummer: **85112075.8**

(22) Anmeldetag: **24.09.85**

(51) Int.Cl.$^4$: **A 61 K 31/55**

(30) Priorität: **01.10.84 DE 3435974**

(43) Veröffentlichungstag der Anmeldung:
**09.04.86 Patentblatt 86/15**

(84) Benannte Vertragsstaaten:
**AT BE CH DE FR GB IT LI LU NL SE**

(71) Anmelder: **BOEHRINGER INGELHEIM KG**

**D-6507 Ingelheim am Rhein(DE)**

(84) Benannte Vertragsstaaten:
**BE CH DE FR IT LI LU NL SE AT**

(71) Anmelder: **Boehringer Ingelheim International G.m.b.H**

**D-6507 Ingelheim am Rhein(DE)**

(84) Benannte Vertragsstaaten:
**GB**

(72) Erfinder: **Casals-Stenzel, Jorge, Dr.**
**Sartoriusring 295**
**D-6500 Mainz 21(DE)**

(72) Erfinder: **Weber, Karl-Heinz, Dr.**
**Kaiser-Karl-Strasse 11**
**D-6535 Gau-Algesheim(DE)**

(72) Erfinder: **Walther, Gerhard, Dr.**
**Pfarrer-Heberer-Strasse 37**
**D-6530 Bingen am Rhein(DE)**

(72) Erfinder: **Harreus, Albrecht, Dr.**
**Sandstrasse 1**
**D-6507 Ingelheim am Rhein(DE)**

(72) Erfinder: **Muacevic, Gojki, Dr.**
**In der Dörrwiese 13**
**D-6507 Ingelheim am Rhein(DE)**

(54) **Diazepine enthaltende pharmazeutische Zusammensetzungen mit Plättchen aktivierender Faktor (PAF)-antagonistischer Wirkung.**

(57) Der Plättchen aktivierende Faktor (Acetylglyceryl-ester-phosphorylcholin, PAF) ist als potenter Lipidmediator bekannt, der von tierischen und menschlichen proinflammatorischen Zellen freigesetzt wird.

Die Erfindung betrifft PAF-antagonistische pharmazeutische Zusammensetzungen, die als Wirkstoff eines oder mehrere Diazepine der allgemeinen Formel I gemäß Anspruch 1 enthalten.

Diese pharmazeutischen Zusammensetzungen dienen zur Behandlung pathologischer Zustände und Krankheiten, and denen PAF beteiligt ist.

EP 0 176 928 A2

Croydon Printing Company Ltd

Die Erfindung betrifft Diazepine enthaltende pharmazeutische Zusammensetzungen mit PAF-antagonistischer Wirkung. Ein großer Teil der Diazepine ist bereits bekannt, andere Diazepine sind neu.

Bei PAF (Plättchen aktivierender Faktor) handelt es sich um das Acetylglycerylester-phosphorylcholin (AGEPC) der Formel

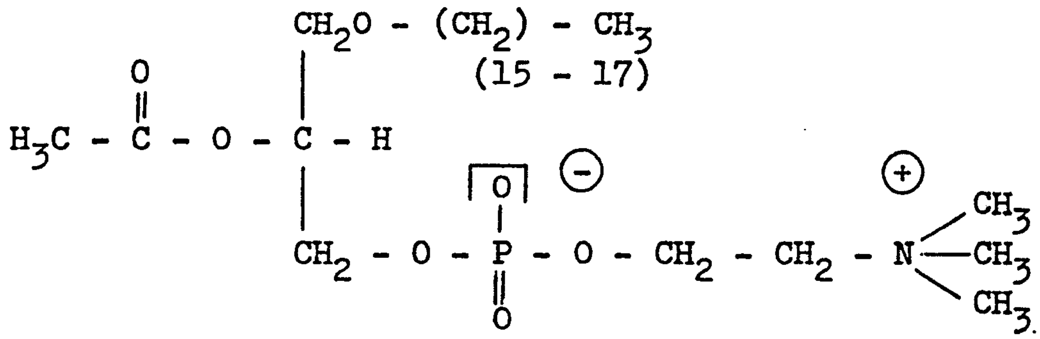

Diese Verbindung ist als potenter Lipidmediator bekannt, der von tierischen und menschlichen proinflammatorischen Zellen freigesetzt wird. Unter solchen Zellen finden sich hauptsächlich basophile und neutrophile Granulozyten, Makrophagen (aus Blut oder Gewebe) sowie Thrombozyten, die an Entzündungsreaktionen beteiligt sind.

PAF zeigt im pharmakologischen Experiment folgende Wirkungen:

a) Bronchokonstriktion, die etwa hundertmal stärker ist als die des Histamins;
b) Blutdrucksenkung, die wahrscheinlich auf eine direkte periphere Vasodilatation zurückzuführen ist;
c) Auslösung einer Thrombozytenaggregation (in vitro und in vivo nachgewiesen);
d) proinflammatorische Wirkung durch Adhäsion und Aggregation von Neutrophilen, gefolgt von einer Freisetzung lysosomaler Enzyme und Aktivierung des Arachidonsäure-Stoffwechsels (in vitro geprüft).

Diese experimentell nachweisbaren Wirkungen des PAF weisen direkt oder indirekt auf mögliche Funktionen dieses Mediators in der Anaphylaxie, in der Pathophysiologie des Asthma bronchiale und in der Entzündung hin.

PAF-Antagonisten werden benötigt, um einerseits weitere pathophysiologische Funktionen dieses Mediators an Tier und Mensch aufzuklären und andererseits pathologische Zustände und Krankheiten, an denen PAF beteiligt ist, zu behandeln. Beispiele für die Indikationen eines PAF-Antagonisten sind Entzündungsprozesse des Tracheobronchialbaumes, akute und chronische Bronchitis, Asthma bronchiale, anaphylaktische Zustände, Allergien sowie Entzündungen im Bereich der Schleimhäute und der Haut.

Es sind bereits Substanzen mit PAF-antagonistischer Wirkung bekannt geworden, zum Beispiel:

1. Substanzen, deren Strukturen sich am PAF orientieren (europäische Patentanmeldung Nr. 94586; US-Patent Nr. 4 329 302; japanische Patentanmeldungen Nr. 57165394, 58035116, 58154512);
2. 11-Oxopyridochinazoline (europäische Patentanmeldung Nr. 94080).

Darüber hinaus wurden Verbindungen aus den folgenden Indikationsgebieten auf PAF-antagonistische Wirkung hin untersucht:

Calciumantagonisten
Antiallergika
Entzündungshemmer
α-Andrenergika

Überraschenderweise wurde nun gefunden, daß zahlreiche Stoffe aus einer bisher nicht berücksichtigten Verbindungsklasse, der der Diazepine, eine starke PAF-antagonistische Wirkung aufweisen.

In den vergangenen zwanzig Jahren sind tausende Molekülvarianten bei Diazepinen synthetisiert und pharmakologisch, biochemisch und zum Teil auch klinisch geprüft worden. Die meisten Diazepine, insbesondere die aus der 1,4-Reihe, wirken anticonvulsiv, anxiolytisch, muskelrelaxierend und mehr oder weniger stark sedativ (S. Garratini et al. "The Benzodiazepines", Raven Press, New York, 1973).
Unter der Vielzahl von Strukturen gibt es einige Ausnahmen, deren Wirkungsprofil ein anderes Bild zeigt. So sind Diazepine mit Wirkung gegen Bilharziose (Drugs of the future 5, 43 (1980)) und gegen hohen Blutdruck (europäische Patentanmeldung Nr. 87850) bekanntgeworden. Bei anderen Diazepinen wurde eine analgetische Wirkung (D. Römer et al. Nature 298, 759 (1982)) und eine Affinität zum Dopaminrezeptor (Ruhland und Liepmann, C.I.N.P.-Kongress, Nürnberg (1983)) festgestellt.
Die PAF-antagonistische Wirkung von Diazepinen ist bisher nicht bekanntgeworden.

Gegenstand der Erfindung sind daher pharmazeutische Zusammensetzungen mit PAF-antagonistischer Wirkung, enthaltend als Wirkstoff eine oder mehrere Verbindungen der allgemeinen Formel

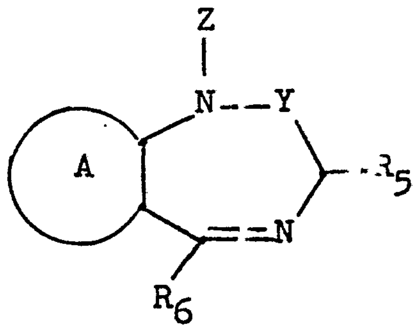

(I)

In dieser Formel bedeuten:

A einen anellierten Ring der nachstehenden Formel

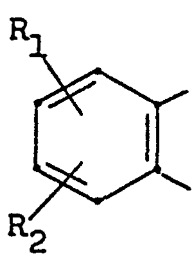
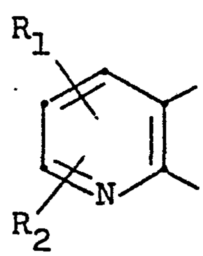
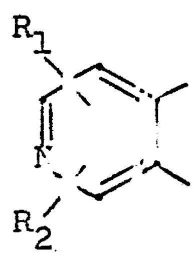
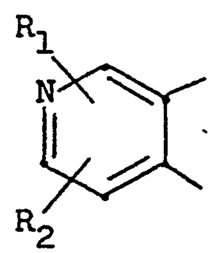
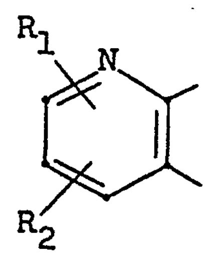

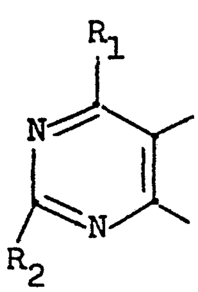
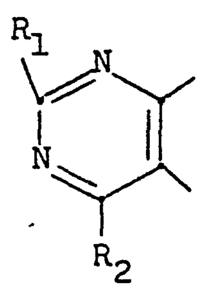
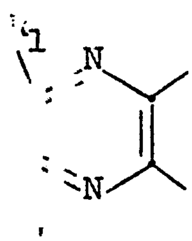

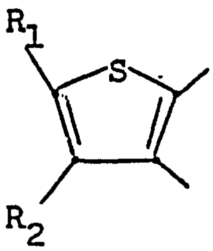 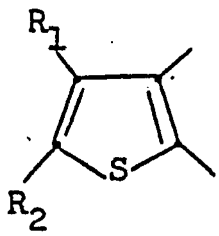 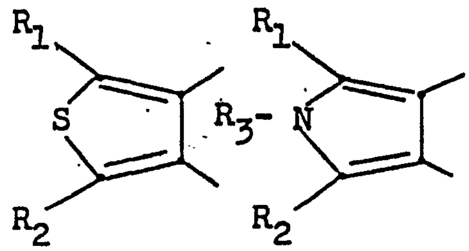

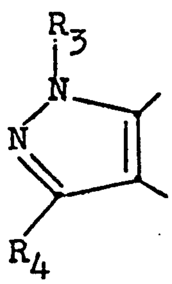 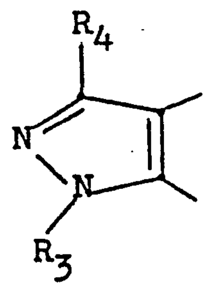 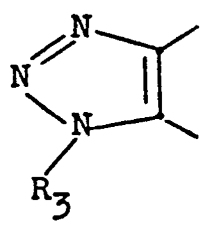 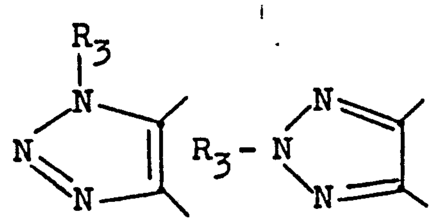

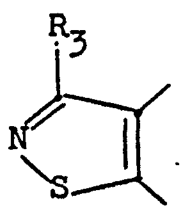 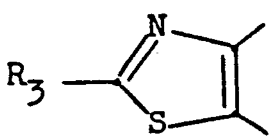 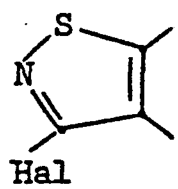 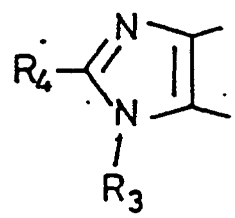

$R_1$ und $R_2$, die gleich oder verschieden sein können, Wasserstoff, eine geradkettige oder verzweigte Alkyl-, Alkenyl- oder Alkinylgruppe mit 1 - 8 Kohlenstoffatomen, wobei diese Gruppe gegebenenfalls ein- oder mehrfach durch Halogen, Hydroxy, Alkoxy, Alkylmercapto, Amino, Alkylamino, Dialkylamino, Alkylcarbonyl, Alkyloxycarbonyl oder eine Säureamidgruppe weiter substituiert sein kann;
einen ankondensierten gesättigten carbocyclischen oder heterocyclischen Ring, wobei letzterer Sauerstoff, Schwefel oder Stickstoff als Heteroatom enthalten kann und der stickstoffhaltige Ring am Stickstoffatom eine Alkylgruppe tragen kann;

Halogen, Trifluormethyl, Nitro,Cyano,optimal subst. Amino, Alkylmercapto, Alkylcarbonyl, Alkoxy, Alkyloxycarbonyl oder eine Säureamidgruppe;

$R_3$ und $R_4$, die gleich oder verschieden sein können, Wasserstoff, eine geradkettige oder verzweigte Alkyl-, Alkenyl- oder Alkinylgruppe mit 1 - 8 Kohlenstoffatomen, wobei diese Gruppe gegebenenfalls ein- oder mehrfach durch Halogen, Hydroxy, Alkoxy, Alkylmercapto, Amino, Alkylamino, Dialkylamino, Alkylcarbonyl, Alkyloxycarbonyl, Cyano oder eine Säureamidgruppe weiter substituiert sein kann:

$R_5$ Wasserstoff, eine geradkettige oder verzweigte Alkyl-, Alkenyl- oder Alkinylgruppe mit 1 - 8 Kohlenstoffatomen, wobei diese Gruppe gegebenenfalls ein- oder mehrfach durch Halogen, Hydroxy, Alkoxy, Alkylmercapto, Amino, Alkylamino, Dialkylamino, Alkylcarbonyl, Alkyloxycarbonyl oder eine Säureamidgruppe weiter substituiert sein kann;

$R_6$ Phenyl, wobei der Phenylring bevorzugt in 2-Stellung durch Methyl, Methoxy, Halogen, Nitro oder Trifluormethyl substituiert sein kann, Thienyl oder α-Pyridyl;

Y $>CO$, $>CS$ oder $>CH_2$; und

Z Wasserstoff oder eine geradkettige oder verzweigte Alkyl-, Alkenyl- oder Alkinylgruppe mit 1 - 8 Kohlenstoffatomen, wobei diese Gruppe gegebenenfalls ein- oder mehrfach durch Halogen, Hydroxy, Alkoxy, Alkylmercapto, Amino, Alkylamino, Dialkylamino, Alkylcarbonyl, Alkyloxycarbonyl oder eine Säureamidgruppe weiter substituiert sein kann und gegebenenfalls deren Säureadditionssalze.

Bevorzugt sind Verbindungen der allgemeinen Formel I, worin A einen anellierten Ring der nachstehenden Formel

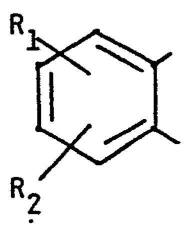 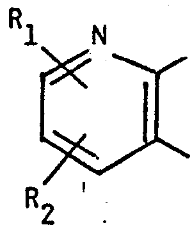 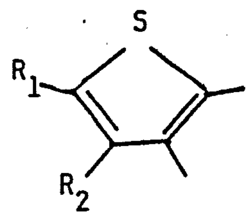 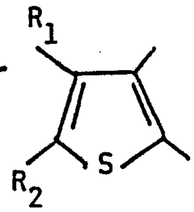 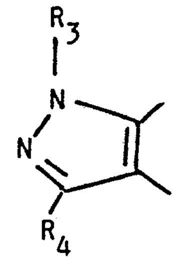

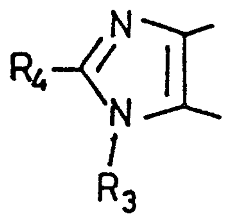

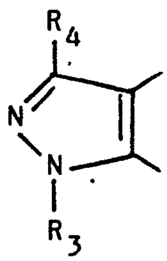 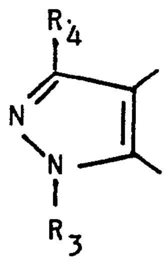 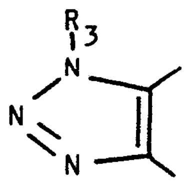 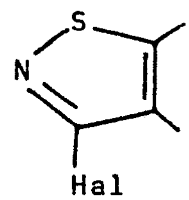

$R_1$ und $R_2$, die gleich oder verschieden sein können, Wasserstoff, eine geradkettige oder verzweigte Alkylgruppe mit 1 - 8 Kohlenstoffatomen; Halogen, wie z.B. F,Cl, Br, eine Nitro-oder eine Alkoxygruppe, Alkyloxycarbonyl,

$R_1$ und $R_2$ zusammen einen heterocyclischen Ring mit wenigstens einem Sauerstoff- oder Schwefelatom;

$R_3$ und $R_4$, die gleich oder verschieden sein können, Wasserstoff, eine geradkettige oder verzweigte Alkylgruppe mit 1 - 8 Kohlenstoffatomen, wobei diese Gruppe mit einer Cyanogruppe weitersubstituiert sein kann,

$R_5$ Wasserstoff, eine geradkettige oder verzweigte Alkylgruppe mit 1 - 8 Kohlenstoffatomen;

| | |
|---|---|
| $R_6$ | eine Phenyl- oder α-Pyridylgruppe; |
| Y | $>C=O$, $>C=S$ oder $>CH_2$; und |
| Z | Wasserstoff oder eine geradkettige oder verzweigte Alkylgruppe mit 1 - 8 Kohlenstoffatomen, wobei diese Gruppe gegebenenfalls mit einer Dialkylaminogruppe weitersubstituiert sein kann, |

bedeuten;
und gegebenenfalls deren Säureadditionssalze.

Bei den vorstehenden Bedeutungen für die Reste $R_1$ bis $R_6$ bedeuten, falls nichts anderes angegeben,
Hal eines der Halogenatome Fluor, Chlor, Brom oder Jod;
Alkyl eine geradkettige oder verzweigte Alkylgruppe mit 1 - 8 Kohlenstoffatomen.
Der Ausdruck "Säureamidgruppe" bedeutet eine am Stickstoff ein- oder zweifach durch Alkyl substituierte Aminocarbonylgruppe; ferner fällt unter diese Bedeutung eine unter Einbeziehung des Stickstoffatoms zum 5- oder 6-Ring geschlossene Aminocarbonylgruppe, wobei der Heteroring gegebenenfalls als weiteres Heteroatom ein Sauerstoff-, Stickstoff- oder Schwefelatom enthalten kann, und wobei das etwa zusätzlich im Ring vorhandene Stickstoffatom eine Alkylgruppe als Substituenten tragen kann.

Die oben angeführten Verbindungen der allgemeinen Formel I sind bekannt oder können nach bekannten Methoden erhalten werden, wie sie beispielsweise beschrieben sind in Jeffrey W. H. Watthey et al "Heterocyclic Compounds" Band 43 (1984), "Azepines" Part 2, Verlag John Wiley & Sons Inc. und L.H. Sternbach "Progress in Drug Research" Vol. 22 (1978), S. 229-263, Birkhäuser Verlag Basel.
Einige der unter die allgemeine Formel I fallende Verbindungen sind befähigt, mit anorganischen oder organischen Säuren Säureadditionssalze zu bilden.
Verbindungen der allgemeinen Formel I, die eine Carbonsäurefunktion enthalten, können als wasserlösliche Alkali- oder Erdalkalisalze erhalten werden.

## Pharmakologische Untersuchungsergebnisse

Die PAF-antagonistische Wirksamkeit von Verbindungen der Formel I wurde anhand der Hemmung der Blutplättchen-Aggregation in vitro und der Antagonisierung der durch PAF bewirkten Bronchokonstriktion am narkotisierten Meerschweinchen untersucht.

1. Hemmung der Blutplättchen-Aggregation in vitro

Zur Bestimmung der PAF-antagonistischen Wirkung von Substanzen wurde die durch PAF induzierte Aggregation von Humanthrombozyten in vitro verwendet.
Zur Gewinnung von thrombozytenreichem Plasma (TRP) erfolgt die Blutentnahme aus einer nicht gestauten Vene mit Hilfe einer Plastikspritze, in der sich 3,8 %ige Natriumcitratlösung befindet. Das Verhältnis zwischen Natriumcitratlösung und Blut beträgt 1:9. Nach vorsichtiger Durchmischung wird das Citratblut bei 150 • g (1200 U/min) 20 Minuten lang zentrifugiert. Die Messung der Thrombozytenaggregation erfolgte nach dem von Born und Cross ausgearbeiteten Verfahren (G. V. R. Born und M. J. Cross, J. Physiol. 168, 178 (1963)), wobei dem TRP unter ständigem Rühren ein Auslöser (PAF) der Aggregation zugesetzt wird.

Die Prüfsubstanz wird jeweils 2 - 3 Minuten vor Auslösung der Aggregation in einem Volumen von 10 µl zugesetzt. Als Lösungsmittel dienen entweder Aqua dest., Äthanol und/oder Dimethylsulfoxyd (jeweils in geeigneter Konzentration).

Kontrollansätze enthalten entsprechende Volumina dieser Lösungsmittel. Nach Registrierung der Ausgangsabsorption (2 - 3 Minuten) wird die Aggregation mit PAF ($5 \times 10^{-8}$M) induziert.

Zur Beurteilung von Substanzeffekten wird das Maximum der ersten Aggregationswelle verwendet. Die durch PAF induzierte maximale Absorptionsrate (≙ maximale Aggregation x 100 %) wird jeweils gleichzeitig in einem Parallelansatz (= Kontrollansatz in einem der Kanäle des 2 Kanal-Aggregometers) zu jedem Testansatz (zweiter Kanal) mitgeprüft und als 100 %-Wert verwendet.

Der unter dem Einfluß der Testsubstanz erreichte Aggregationswert wird als % des Kontrollwertes - bei einer Aggregationshemmung unter 100 %, bei einer Steigerung über 100 % - angegeben.

Jede Prüfsubstanz wird bei Konzentrationen von $10^{-3}$ bis $10^{-8}$ M mit einem Stichprobenumfang von jeweils n = 4 hinsichtlich einer hemmenden Wirkung auf die durch PAF induzierte Thrombozytenaggregation untersucht. Danach wird eine Konzentrations-Wirkungskurve anhand von 3 Konzentrationen erstellt und die $IK_{50}$ (Konzentration bei einer 50%igen Aggregationshemmung) ermittelt.

2. Antagonisierung der durch PAF bewirkten Bronchokonstriktion an narkotisierten Meerschweinchen

Spontan atmende männliche, 300 - 450 g schwere Meerschweinchen werden 1 Stunde vor der i.v.-Infusion mit PAF (30 mg/kg x min) mit der Testsubstanz oder einem Kontrollvehikel oral vorbehandelt. Die Versuchstiere werden dann mit 2 mg/kg Urethan intraperitoneal

anästhetisiert, worauf man die Vena jugularis, die Aorta carotis und die Trachea kanüliert. Die PAF-Infusion induziert bei den Kontrolltieren eine starke, anhaltende Bronchokonstriktion, die anhand des Atemwegvolumens, der Compliance und der Resistance gemessen wird, ebenso eine Senkung des Blutdruckes. Nach ca. 7 - 10 Minuten tritt der Tod ein.
Mit den beschriebenen PAF-Antagonisten können diese Effekte auf Atmung und Blutdruck sowie der Eintritt des Todes verhindert werden. Die dafür erforderlichen Dosen bewegen sich zwischen 1 und 50 mg/kg p.o. und 0,1 bis 1,0 mg/kg i.v..

In der folgenden Tabelle ist die $IK_{50}$ einer Reihe von Verbindungen der allgemeinen Formel I angegeben:

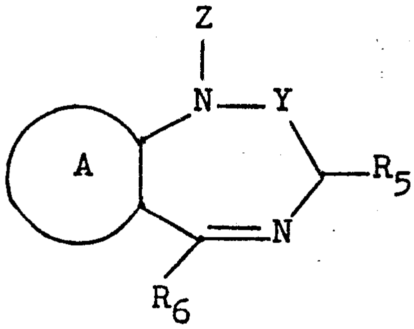

| Nr. | A | Z | $R_5$ | Y | $R_6$ | PAF-Antag. $IK_{50}$; [µMol] | Fp. °C |
|---|---|---|---|---|---|---|---|
| 1 Diazepam | 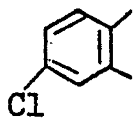 | $CH_3$ | H | >CO | 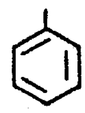 | 250 | 128 - 130 |
| 2 Nitraze-pam | 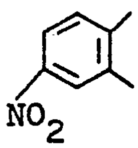 | H | H | >CO | 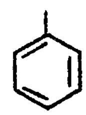 | 220 | 228 - 230 |
| 3 Clonaze-pam | 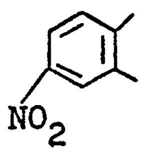 | H | H | >CO | 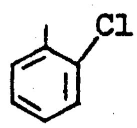 | 80 | 236 - 238 |
| 4 Flunitra-zepam | 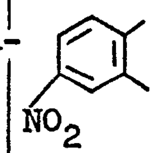 | $CH_3$ | H | >CO | 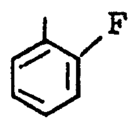 | 46 | 169 - 170 |
| 5 Fluraze-pam Dihydro-chlorid | 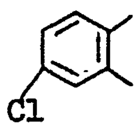 | $N(C_2H_5)_2$<br>\|<br>$(CH_2)_2$<br>\| | H | >CO | 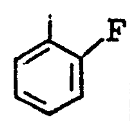 | 80 | 218 - 220 |
| 6 Brom-azepam | 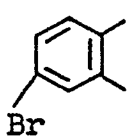 | $CH_3$ | H | >CO | 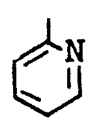 | 1000 | 234 - 238 |
| 7 | 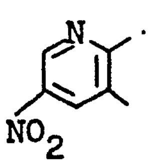 | $CH_3$ | H | >CO | 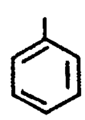 | 380 | 221 - 222 |

| Nr. | A | Z | $R_5$ | Y | $R_6$ | PAF-Antag. $IK_{50}$;[μMol] | Fp. °C |
|---|---|---|---|---|---|---|---|
| 8 | 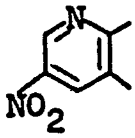 | H | H | 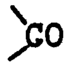 | 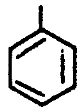 | 250 | 224 |
| 9 | 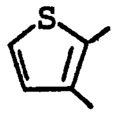 | H | H | 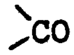 | 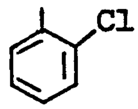 | 760 | 231 - 233 |
| 10 | 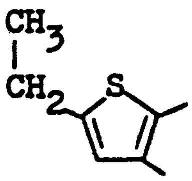 | H | H | 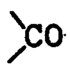 | 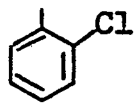 | 325 | 210 - 212 |
| 11 | 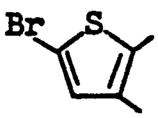 | $CH_3$ | H | 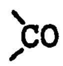 | 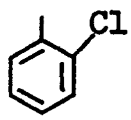 | 170 | 112 - 114 |
| 12 | 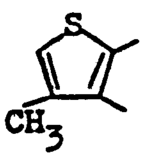 | H | H | 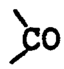 | 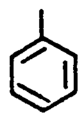 | 280 | 253 - 254 |
| 13 | 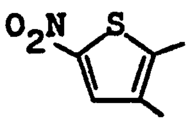 | $CH_3$ | H | 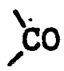 | 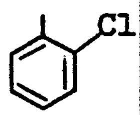 | 200 | 160 - 161 |
| 14 | 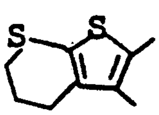 | H | H | 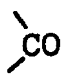 | 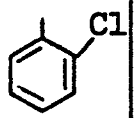 | 234 | 225 - 227 |
| 15 | 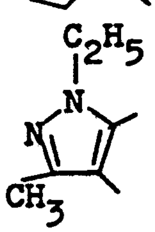 | H | H | 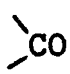 | 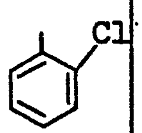 | 870 | 255 - 256 |

| Nr. | A | Z | $R_5$ | Y | $R_6$ | PAF-Antag. $IK_{50}$;[µMol] | Fp. °C |
|---|---|---|---|---|---|---|---|
| 16 | 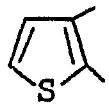 | H | H | CO | 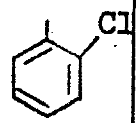 | 500 | 222 - 224 |
| 17 | 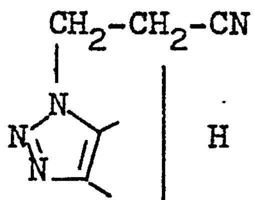 | H | H | CO | 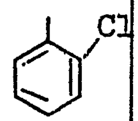 | 1000 | 231 - 235 |
| 18 | 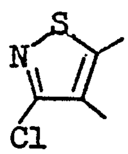 | $CH_3$ | H | $CH_2$ | 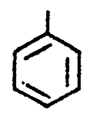 | 110 | 205 - 208 |
| 19 | 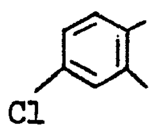 | H | H | C=S | 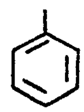 | 620 | 240 - 245 |
| 20 | 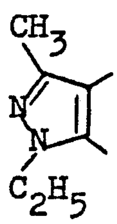 | H | H | C=S | 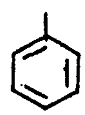 | 250 | 280 - 281 |
| 21 | 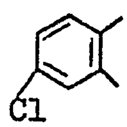 | $CH_3$ | H | $CH_2$ | 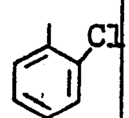 | 360 | 190 - 192 |
| 22 | 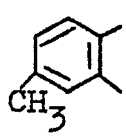 | H | H | CO | 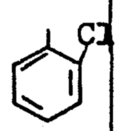 | 580 | 226 - 228 |
| 23 | 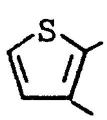 | H | $C_2H_5$ | CO | 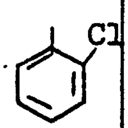 | 810 | 126 - 128 |
| 24 | 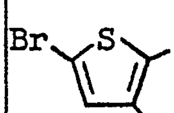 | H | $C_2H_5$ | CO | 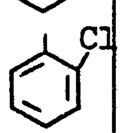 | 530 | 234 - 236 |

| Nr. | A | Z | $R_5$ | Y | $R_6$ | PAF-Antag. $IK_{5o}$;[µMol] | Fp.°C |
|---|---|---|---|---|---|---|---|
| 25 | 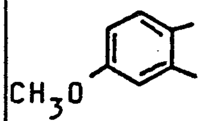 | H | H | >CO | 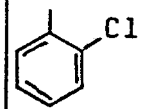 | > 1oo | 22o-223 |
| 26 | 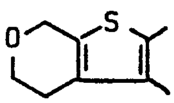 | H | H | >CO | 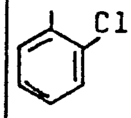 | 79 | 225-227 |
| 27 | 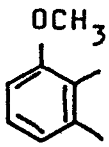 | H | H | >CO | 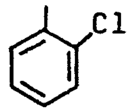 | 356 | 167 |
| 28 | 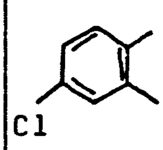 | H | H | >CO | 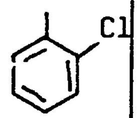 | > 1oo | 197-198 |
| 29 | 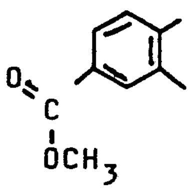 | H | H | >CO | 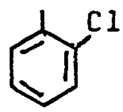 | 12o | 255-256 |
| 3o | 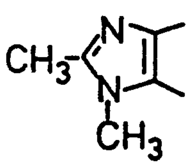 | H | H | >CO | 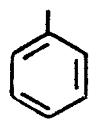 | > 5o | 334-335 |

Die für die neue Indikation anwendbaren Verbindungen der allgemeinen Formel I können warmblütigen Tieren topisch, oral, parenteral oder durch Inhalation verabreicht werden. Die Verbindungen liegen hierbei als aktive Bestandteile in üblichen Darreichungsformen vor, z.B. in Zusammensetzungen, die im wesentlichen aus einem inerten pharmazeutischen Träger und einer effektiven Dosis des Wirkstoffes bestehen, wie z.B. Tabletten, Dragees, Kapseln, Oblaten, Pulver, Lösungen, Suspensionen, Inhalationsaerosole, Salben, Emulsionen, Sirupe, Suppositorien usw.. Eine wirksame Dosis der erfindungsgemäßen Verbindungen liegt bei oraler Anwendung zwischen 10 und 500, vorzugsweise zwischen 25 und 100 mg pro Dosis, bei intravenöser oder intramuskulärer Anwendung zwischen 0,01 und 100, vorzugsweise zwischen 0,1 und 50 mg pro Dosis. Für die Inhalation sollen Lösungen, die 0,01 bis 1,0, vorzugsweise 0,1 bis 0,5 % Wirkstoff enthalten, eingesetzt werden.

Nachfolgend werden Beispiele für einige pharmazeutische Zusammensetzungen unter Verwendung von Verbindungen der allgemeinen Formel I als aktiver Bestandteil angegeben.
Falls nicht ausdrücklich anders bezeichnet, handelt es sich bei den Teilen um Gewichtsteile.

1. Tabletten

Die Tablette enthält folgende Bestandteile:

| | | |
|---|---|---|
| Wirkstoff gemäß Formel I | | 0,050 Teile |
| Stearinsäure | | 0,010 " |
| Dextrose | | 1,890 " |
| | gesamt | 1,950 Teile |

Herstellung:

Die Stoffe werden in bekannter Weise zusammengemischt und die Mischung zu Tabletten verpreßt, von denen jede 1,95 g wiegt und 10 - 50 mg Wirkstoff enthält.

2. Salbe

Die Salbe setzt sich aus folgenden Bestandteilen zusammen:

| | | |
|---|---|---|
| Wirkstoff gemäß Formel I | 2,000 | Teile |
| Natriumpyrosulfit | 0,050 | " |
| Mischung (1:1) von Cetylalkohol und Stearylalkohol | 20,000 | " |
| Weiße Vaseline | 5,000 | " |
| Synthetischem Bergamottöl | 0,075 | " |
| Destilliertes Wasser | ad 100,000 | " |

Herstellung:

Die Bestandteile werden in an sich bekannter Weise innig zu einer Salbe vermischt, von der 100 g 2,0 g der Wirksubstanz enthalten.

3. Inhalationsaerosol

Das Aerosol ist aus folgenden Bestandteilen zusammengesetzt:

| | | |
|---|---|---|
| Wirkstoff gemäß Formel I | 1,00 | Teile |
| Sojalecithin | 0,20 | " |
| Treibgasmischung (Frigen® 11, 12 und 14) | ad 100,00 | " |

Herstellung:

Die Bestandteile werden in an sich bekannter Weise zusammengemischt und das Gemisch in Aerosolbehälter gefüllt, die ein Dosierventil enthalten, das pro Betätigung zwischen 1 und 20 mg Wirksubstanz abgibt.

4. Ampullenlösung

Die Lösung setzt sich aus folgenden Bestandteilen zusammen:

| | | |
|---|---|---|
| Wirkstoff gemäß Formel I | 5,0 | Teile |
| Polyethylenglykol | 400,0 | " |
| Benzylalkohol | 15,0 | " |
| Ethylalkohol (95 %) | 100,0 | " |
| Natriumbenzoat | 50,0 | " |
| Benzoesäure | 1,2 | " |
| Doppelt destilliertes Wasser | ad 1000,0 | " |

Herstellung:
Der Wirkstoff wird in Benzylalkohol gelöst und danach Polyethylenglykol mit Alkohol zugesetzt. Natriumbenzoat und Benzoesäure werden in 250 ml Wasser gelöst, mit obiger Lösung vereinigt und mit Wasser auf 1000 ml aufgefüllt. Die erhaltene Lösung wird pyrogenfrei filtriert und das Filtrat unter aseptischen Bedingungen in 1 ml Ampullen abgefüllt, die anschließend sterilisiert und zugeschmolzen werden. Jede Ampulle enthält 1 - 5 mg des Wirkstoffs.

5. Suppositorien

Jedes Zäpfchen enthält:

| | | |
|---|---|---|
| Wirkstoff gemäß Formel I | 1,0 | Teile |
| Kakaobutter (Fp. 36 - 37°C) | 1200,0 | " |
| Carnaubawachs | 5,0 | " |

Herstellung:

Kakaobutter und Carnaubawachs werden zusammengeschmolzen. Bei 45°C gibt man den Wirkstoff hinzu und rührt bis eine komplette Dispersion entstanden ist.
Die Mischung wird in Formen entsprechender Größe gegossen und die Zäpfchen zweckmäßig verpackt.

## P a t e n t a n s p r ü c h e

1. Verwendung von Verbindungen der allgemeinen Formeln

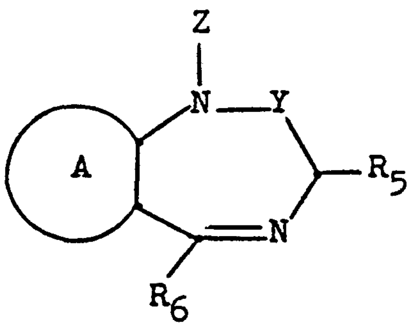

(I)

**worin**

A einen anellierten Ring der nachstehenden Formel

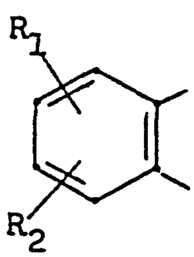
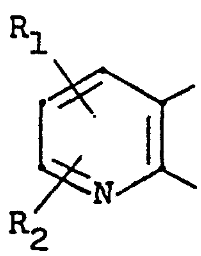
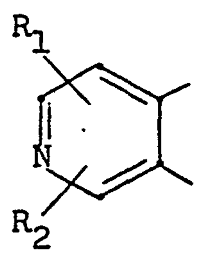
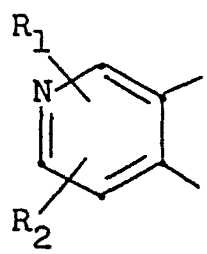
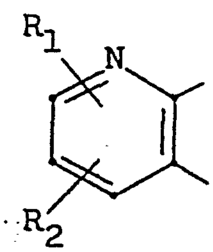

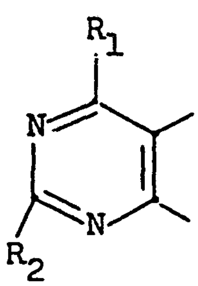
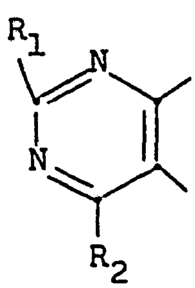
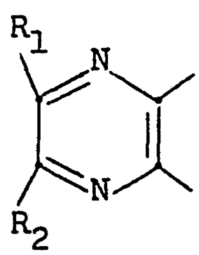

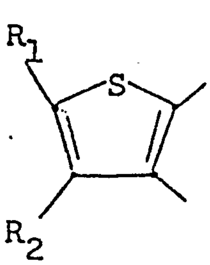
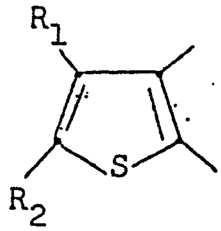
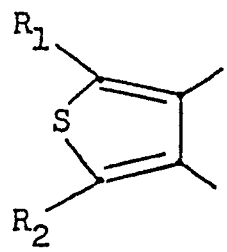
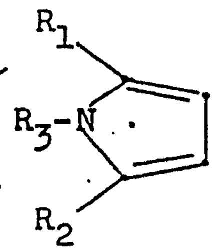

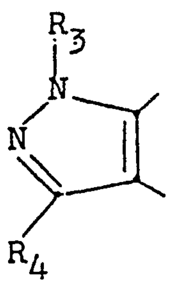
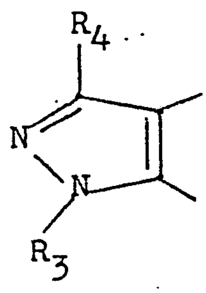
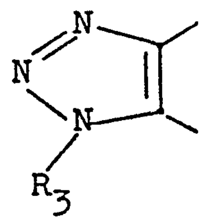
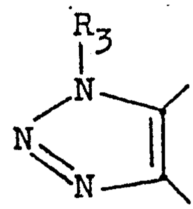
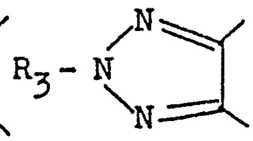

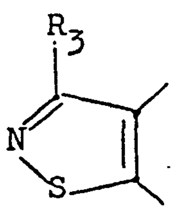
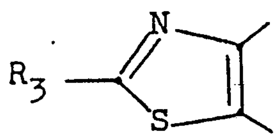
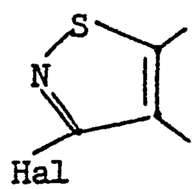
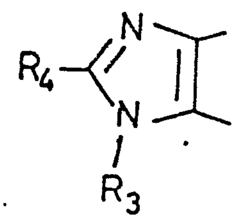

$R_1$ und $R_2$, die gleich oder verschieden sein können, Wasserstoff, eine geradkettige oder verzweigte Alkyl-, Alkenyl- oder Alkinylgruppe mit 1 - 8 Kohlenstoffatomen, wobei diese Gruppe gegebenenfalls ein- oder mehrfach durch Halogen, Hydroxy, Alkoxy, Alkylmercapto, Amino, Alkylamino, Dialkylamino, Alkylcarbonyl, Alkyloxycarbonyl oder eine Säureamidgruppe weiter substituiert sein kann;

einen ankondensierten gesättigten carbocyclischen oder heterocyclischen Ring, wobei letzterer Sauerstoff, Schwefel oder Stickstoff als Heteroatom enthalten kann und der stickstoffhaltige Ring am Stickstoffatom eine Alkylgruppe tragen kann;

Halogen, Trifluormethyl, Nitro,Cyano,optimal subst. Amino, Alkylmercapto, Alkylcarbonyl, Alkoxy, Alkyloxycarbonyl oder eine Säureamidgruppe;

$R_3$ und $R_4$, die gleich oder verschieden sein können, Wasserstoff, eine geradkettige oder verzweigte Alkyl-, Alkenyl- oder Alkinylgruppe mit 1 - 8 Kohlenstoffatomen, wobei diese Gruppe gegebenenfalls ein- oder mehrfach durch Halogen, Hydroxy, Alkoxy, Alkylmercapto, Amino, Alkylamino, Dialkylamino, Alkylcarbonyl, Alkyloxycarbonyl, Cyano oder eine Säureamidgruppe weiter substituiert sein kann;

$R_5$ Wasserstoff, eine geradkettige oder verzweigte Alkyl-, Alkenyl- oder Alkinylgruppe mit 1 - 8 Kohlenstoffatomen, wobei diese Gruppe gegebenenfalls ein- oder mehrfach durch Halogen, Hydroxy, Alkoxy, Alkylmercapto, Amino, Alkylamino, Dialkylamino, Alkylcarbonyl, Alkyloxycarbonyl oder eine Säureamidgruppe weiter substituiert sein kann;

$R_6$ Phenyl, wobei der Phenylring bevorzugt in 2-Stellung durch Methyl, Methoxy, Halogen, Nitro oder Trifluormethyl substituiert sein kann, Thienyl oder α-Pyridyl;

Y $>CO$, $>CS$ oder $>CH_2$; und

Z Wasserstoff oder eine geradkettige oder verzweigte Alkyl-, Alkenyl- oder Alkinylgruppe mit 1 - 8 Kohlenstoffatomen, wobei diese Gruppe gegebenenfalls ein- oder mehrfach durch Halogen, Hydroxy, Alkoxy, Alkylmercapto, Amino, Alkylamino, Dialkylamino, Alkylcarbonyl, Alkyloxycarbonyl oder eine Säureamidgruppe weiter substituiert sein kann, bedeuten,

und gegebenenfalls deren Säureadditionssalze zur Herstellung eines Arzneimittels zur Behandlung von pathologischen Zuständen und Krankheiten, an denen PAF beteiligt ist.

2. Verwendung von Verbindungen der allgemeinen Formel I nach Anspruch 1, worin A einen anellierten Ring der nachstehenden Formel

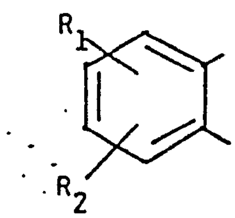
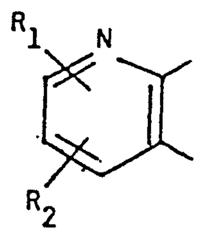
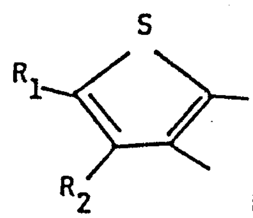
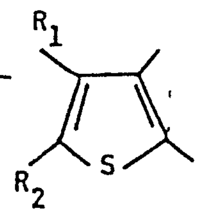
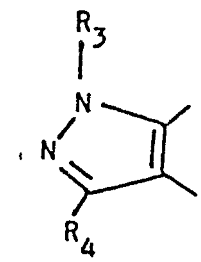

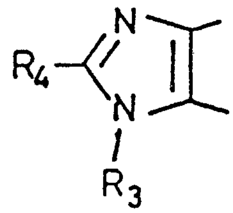

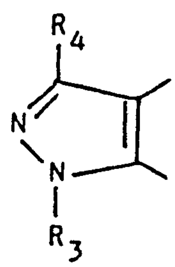
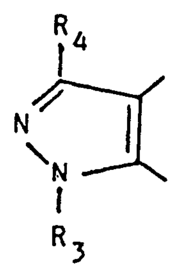
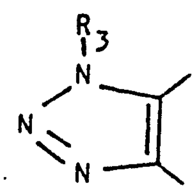
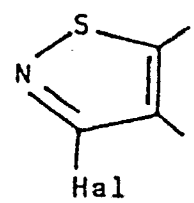

$R_1$ und $R_2$, die gleich oder verschieden sein können, Wasserstoff, eine geradkettige oder verzweigte Alkylgruppe mit 1 - 8 Kohlenstoffatomen; Halogen, wie z.B. F,Cl, Br, eine Nitro-oder eine Alkoxygruppe, Alkyloxycarbonyl,
$R_1$ und $R_2$ zusammen einen heterocyclischen Ring mit wenigstens einem Sauerstoff- oder Schwefelatom;

$R_3$ und $R_4$, die gleich oder verschieden sein können, Wasserstoff, eine geradkettige oder verzweigte Alkylgruppe mit 1 - 8 Kohlenstoffatomen, wobei diese Gruppe mit einer Cyanogruppe weitersubstituiert sein kann,

$R_5$ Wasserstoff, eine geradkettige oder verzweigte Alkylgruppe mit 1 - 8 Kohlenstoffatomen;

$R_6$ eine Phenyl- oder α-Pyridylgruppe;

Y $>C=O$, $>C=S$ oder $>C\ H_2$; und

Z Wasserstoff oder eine geradkettige oder verzweigte Alkylgruppe mit 1 - 8 Kohlenstoffatomen, wobei diese Gruppe gegebenenfalls mit einer Dialkylaminogruppe weitersubstituiert sein kann,

bedeuten,

und gegebenenfalls deren Säureadditionssalze, zur Herstellung eines Arzneimittels zur Behandlung von pathologischen Zuständen und Krankheiten, an denen PAF beteiligt ist.

3. Verwendung von Verbindungen der allgemeinen Formel I nach Anspruch 1 zur Herstellung eines Arzneimittels zur Behandlung von opstruktiven Lungenerkrankungen, wie z.B. Entzündungsprozessen des Tracheobronchialbaumes, akute und chronische Bronchitis und Asthma bronchiale.

4. Verwendung von Verbindungen der allgemeinen Formel I nach Anspruch 1 zur Herstellung eines Arzneimittels zur Behandlung von allergischen entzündlichen Reaktionen, wie z.B. anaphylaktischen Zuständen.

5. Verwendung von Verbindungen der allgemeinen Formel I nach Anspruch 1 zur Herstellung eines Arzneimittels zur Behandlung von Allergien.

6. Verwendung von Verbindungen der allgemeinen Formel I nach Anspruch 1 zur Herstellung eines Arzneimittels zur Behandlung von Entzündungen im Bereich der Schleimhäute und der Haut.

7. Verwendung von Verbindungen der allgemeinen Formel I nach Anspruch 1 zur Herstellung eines Arzneimittels zur Behandlung von Koagulapatien und Thrombosen.

8. Verwendung von Verbindungen der allgemeinen Formel I nach Anspruch 1 zur Herstellung eines Arzneimittels zur Behandlung von Schockzuständen bei Infektionen, Verbrennungen und anderen lebensbedrohlichen Zuständen.